# EUROPEAN PATENT SPECIFICATION

(11) **EP 3 791 874 B1**
(45) Date of publication and mention of the grant of the patent: **24.12.2025**
(21) Application number: 19799633.3
(22) Date of filing: 07.05.2019
(51) Int. Cl.: A61K 31/423, A61K 31/22, A61K 31/366, A61K 31/40, A61K 31/404, A61K 31/47, A61K 31/505, A61K 45/00, A61P 3/06, A61P 9/00, A61P 43/00, A61K 45/06, A61P 9/10

(54) **PEMAFIBRATE AND PITAVASTATIN FOR USE IN REDUCING HYPERTENSION AS A MAJOR RISK FACTOR FOR CARDIOVASCULAR DISEASES**
PEMAFIBRAT UND PITAVASTATIN ZUR VERWENDUNG BEI DER REDUZIERUNG VON BLUTHOCHDRUCK ALS EIN WICHTIGER RISIKOFAKTOR FÜR KARDIOVASKULÄRE ERKRANKUNGEN
PEMAFIBRATE ET PITAVASTATINE POUR UNE UTILISATION DANS LA RÉDUCTION DE L'HYPERTENSION EN TANT QUE FACTEUR DE RISQUE MAJEUR POUR DES MALADIES CARDIOVASCULAIRES

(30) Priority: 08.05.2018 JP 2018089786
(43) Date of publication of application: 17.03.2021
(73) Proprietor: National University Corporation Okayama University, Kita-ku Okayama-shi Okayama 700-8530 (JP); KOWA COMPANY, LTD., Naka-ku Nagoya-shi Aichi 460-8625 (JP)
(72) Inventor: ITO, Hiroshi, Okayama-shi, Okayama 700-8530 (JP); NAKAMURA, Kazufumi, Okayama-shi, Okayama 700-8530 (JP); YOSHIDA, Masatoki, Okayama-shi, Okayama 700-8530 (JP); INOUE, Keisuke, Higashimurayama-shi, Tokyo 189-0022 (JP)
(74) Representative: Wächtershäuser & Hartz Patentanwaltspartnerschaft mbB
(86) International application number: PCT/JP2019/018265
(87) International publication number: WO 2019/216313

(56) References cited:
- US-A1- 2018 028 505
- LAURIE L YEE ET AL: "Pitavastatin Calcium: Clinical Review of a New Antihyperlipidemic Medication", CLINICAL THERAPEUTICS, ELSEVIER, AMSTERDAM, NL, vol. 33, no. 8, 14 June 2011 (2011-06-14), pages 1023 - 1042, XP028268223, ISSN: 0149-2918, [retrieved on 20110721], DOI: 10.1016/J.CLINTHERA.2011.07.011
- YOSHIDA M ET AL: "Combination therapy with pemafibrate (K-877) and pitavastatin improves vascular endothelial function in DAHL/salt-sensitive rats", CIRCULATION, vol. 138, no. Suppl 1, November 2018 (2018-11-01), pages A11905, XP055879729
- OYAMA JUN-ICHI ET AL: "Do antilipidemic agents reduce blood pressure?", VASCULAR FAILURE, vol. 2, no. 1, 31 March 2018 (2018-03-31), pages 6 - 10, XP093135310, ISSN: 2432-4477, DOI: 10.30548/vascfail.2.1_6
- ARAI, HIDENORI ET AL.: "Efficacy and safety of K-877, a novel selective peroxisome proliferator-activated receptor a modulator(SPPARM a), in combination with statin treatment: Two randomised, double-blind, placebo-controlled clinical trials in patients with dyslipidaemia", ATHEROSCLEROSIS, vol. 261, June 2017 (2017-06-01), pages 144 - 152, XP085023909, DOI: 10.1016/j.atherosclerosis.2017.03.032
- PRADHAN, ARUNA D. ET AL.: "Rationale and design of the Pemafibrate to Reduce Cardiovascular Outcomes by Reducing Triglycerides in Patients with Diabetes (PROMINENT) study", AMERICAN HEART JOURNAL, vol. 206, December 2018 (2018-12-01), pages 80 - 93, XP085555556, DOI: 10.1016/j.ahj.2018.09.011

## Description

### TECHNICAL FIELD

The present invention relates to medicament useful for cardiovascular diseases.

### BACKGROUND ART

Cardiovascular diseases (CVD) are a group of diseases mainly caused by vascular disorders such as arteriosclerosis. Examples of the CVD include heart and circulatory disorders including coronary artery disease, cerebrovascular disease, peripheral arterial disease, rheumatic heart disease, congenital heart disease, deep venous thrombosis and pulmonary embolism.

Despite significant advances in health care, CVD remains the leading cause of death worldwide, with an estimated 17.5 million people who were killed by CVD in 2012, which accounts for 31% of all deaths worldwide.
As main risk factors of CVD, hypertension, dyslipidemia, obesity, smoking and the like are known.

Above all, dyslipidemia is an imbalance in human lipid metabolism, one or more lipid values of which are associated with an increased risk of CVD.
Dyslipidemia is well known to be seen in patients with type 2 diabetes mellitus and other patients at high risk for CVD.

Dyslipidemia is typically expressed as one or a combination of an increase in the level of low-density lipoprotein cholesterol (LDL-C), an increase in the level of triglyceride (TG), or a decrease in the level of high-density lipoprotein cholesterol (HDL-C).

Pharmacotherapy targeting at treating dyslipidemia has been regarded as important in order to mitigate the risk of CVD.

According to Non-Patent Literature 1, the following values are reported as cutpoints for starting the treatment of dyslipidemia, i.e. various lipid concentrations at risk for cardiovascular events.

That is, LDL-C > 100 mg/dL (2.59 mmol/L), total cholesterol > 200 mg/dL (5.18 mmol/L), HDL-C <40 mg/dL (1.0 mmol/L) in men and HDL-C < 50 mg/dL (1.3 mmol/L) in women, fasting TGs > 150 mg/dL (1.70 mmol/L), and non-HDL-C > 130 mg/dL (3.37 mmol/L).

Statins, known as HMG-CoA reductase inhibitors, are widely used worldwide for the treatment of dyslipidemia. This class of drugs is believed to be particularly effective in reducing LDL-C levels and reducing the prevalence of CVD.

However, statins have limited effects on cardiovascular risks other than high LDL-C; such as increase in TG, increase in non-HDL-C, increase in apolipoprotein CIII (Apo CIII), increase in remnant cholesterol, and decrease in HDL-C.

Other drug classes, especially fibrates, have also been used to correct lipid imbalances.
Fibrates are particularly effective in activating peroxisome proliferator-activated receptor α (PPARα) and reducing triglycerides and cholesterol.

Fibrates have been used to treat hypertriglyceridemia and hypercholesterolemia, alone or in combination with other lipid ameliorating therapy, and the effectiveness for reducing the risk of CVD has been validated in several large-scale clinical trials.

In FIELD study (Non-Patent Literature 2), 9,795 patients aged 50-75 years with type 2 diabetes were treated with placebo or fenofibrate for five years.
The results showed that fenofibrate administration did not show efficacy on a coronary event which is a primary endpoint, although it showed efficacy on secondary endpoints such as non-fatal myocardial infarction and coronary reconstruction.

In addition, in ACCORD study (Non-Patent Literature 3), the results of simvastatin alone or simvastatin in combination with fenofibrate were followed for a mean of 4.7 years in 5518 patients with type 2 diabetes. No significant additional therapy effects of fenofibrate were detected in any of the primary and secondary endpoints among fatal cardiovascular events, non-fatal myocardial infarction, and non-fatal stroke.

Under these circumstances, further efforts are still ongoing toward the treatment of CVD.

Patent Literature 1 relates to a method of preventing an adverse cardiovascular event in a patient with type 2 diabetes mellitus comprising administering to the patient therapeutically effective amounts of pemafibrate and a statin.

Non-Patent Literature 4 relates to the efficacy and safety of K-877, a selective peroxisome proliferator-activated receptor α modulator (SPPARMα) in combination with statin treatment.

### PRIOR ART LITERATURES

### NON-PATENT LITERATURES

Non-Patent Literature 1: Circulation 2002 Dec 17; 106 (25): 3143-421
Non-Patent Literature 2: LANCET 2005; 366: 1849-1861
Non-Patent Literature 3: N ENGL J MED 2010; 362: 1563-1574
Non-Patent Literature 4: Atherosclerosis, 2017, vol. 261, 144-152

### PATENT LITERATURES

Patent Literature 1 : US 2018/028505

### SUMMARY OF THE INVENTION

### PROBLEM TO BE SOLVED BY THE INVENTION

The present invention relates to providing a medicament useful for cardiovascular diseases.

### MEANS FOR SOLVING THE PROBLEM

Under the above circumstances, the present inventors carried out intensive studies to find that a pemafibrate ((2R)-2-[3-({1,3-benzoxazol-2-yl[3-(4-methoxyphenoxy)propyl]amino}methyl)phenoxy]butanoic acid) known as a selective PPARα modulator (SPPARMα) in combination with the HMG-CoA reductase inhibitor pitavastatin exhibits excellent preventive/therapeutic effect against a cardiovascular disease, to complete the present invention.

The present invention relates to the combination of pemafibrate or a salt thereof and pitavastatin, a salt thereof or a solvate thereof for the uses as defined in the appended claims 1 to 3.

### ADVANTAGEOUS EFFECT OF THE INVENTION

The present invention provides a novel agent for prevention and/or treatment of a cardiovascular disease, which exhibits an excellent reducing cardiovascular risks by combining these two drugs.

### BRIEF DESCRIPTION OF THE DRAWINGS

FIG. 1 shows a diagram showing hypotension when pemafibrate and pitavastatin were administered alone or in combination to Dahl salt-sensitive rats under conditions of high-concentration salt and high-fat diet.
FIG. 2 is a diagram showing heart weights when pemafibrate and pitavastatin were administered alone or in combination to Dahl salt-sensitive rats under conditions of high-concentration salt and high-fat diet.
FIG. 3 is a diagram showing vascular endothelial function when pemafibrate and pitavastatin were administered alone or in combination to Dahl salt-sensitive rats under conditions of high-concentration salt and high-fat diet.
Fig. 4 is a diagram showing the effect of pemafibrate and pitavastatin administered alone or in combination on the expression amount of phosphorylated endothelial nitric oxide synthase (Phospho-eNOS).

### MODES FOR CARRYING OUT THE INVENTION

The definitions of the terms herein are as follows. Note that all technical and scientific terms used herein have the same meaning as commonly understood by a person skilled in the art of the technical field to which the present invention belongs, unless otherwise specified.

As used herein, "pemafibrate" means (2R)-2-[3-({1,3-benzoxazol-2-yl[3-(4-methoxyphenoxy)]propylamino}methyl)phenoxy]butanoic acid, and can be produced by the method described in the pamphlet of International Publication No. WO 2005/023777. Pemafibrate is also available as "Parmodia (registered trademark) tablet 0.1 mg" which is approved in Japan as a therapeutic drug for hyperlipidemia (including familial hyperlipidemia). The salts of pemafibrate include pharmaceutically acceptable salts, for example, acid addition salts such as hydrochloride and basic salts such as sodium salts and potassium salts.

The dose of pemafibrate or a salt thereof is not limited specifically, and can be appropriately examined and determined according to the sex, age, symptoms, and the like of the recipient. For example, it is preferable to take from 0.001 to 100 mg, more preferably from 0.01 to 10 mg, and particularly preferably from 0.1 to 0.4 mg, in terms of the free form of pemafibrate, once or more per day.

A "HMG-CoA reductase inhibitor" is a statin, and examples thereof include pravastatin, simvastatin, fluvastatin, atorvastatin, pitavastatin, rosuvastatin, a salt thereof or a solvate thereof. The present invention concerns the HMG-COA reductase inhibitor pitavastatin, a salt thereof or a solvate thereof. In addition, pravastatin, simvastatin, fluvastatin, atorvastatin, pitavastatin, rosuvastatin, a salt thereof or a solvate thereof are available as commercial products.

As used herein, "pitavastatin, a salt thereof or a solvate thereof" includes pitavastatin itself, but also pharmaceutically acceptable salts of pitavastatin (alkali metal salts such as a sodium salt and a potassium salt; alkaline earth metal salts such as a calcium salt and a magnesium salt; organic amine salts such as a phenethylamine salt; ammonium salts, and the like), as well as solvates of pitavastatin or the pharmaceutically acceptable salts thereof with water, alcohol, or the like, and one or more of these can be used in combination.

As pitavastatin, a salt thereof or a solvate thereof, a calcium salt of pitavastatin or a hydrate thereof is preferable, and pitavastatin calcium (chemical name: (+)-monocalcium bis[(3R,5S,6E)-7-[2-cyclopropyl-4-(4-fluorophenyl)-3-quinolyl]-3,5-dihydroxy-6-heptenoate]) or a hydrate thereof (particularly pentahydrate) is particularly preferable.

Pitavastatin, a salt thereof or a solvate thereof is a known compound and can be produced, for example, by the methods described in JP-A-H1-279866, U.S. Patent No. 5856336 and the like.

The dose of pitavastatin, a salt thereof or a solvate thereof is not limited, and can be appropriately examined and determined according to the sex, age, symptoms, and the like of the recipient. For example, it is preferable to take from 0.1 to 16 mg, more preferably from 0.5 to 8 mg, and particularly preferably from 1 to 4 mg, in terms of pitavastatin calcium, once or more per day.

As the HMG-CoA reductase inhibitor, pitavastatin, a salt thereof or a solvate thereof is used in the invention from the viewpoint of obtaining an excellent cardiovascular risk reducing effect when combined with pemafibrate or a salt thereof. As will be apparent from the Examples described below, a synergistic enhancement of the cardiovascular risk reducing effect such as blood pressure reduction, can be obtained by using pitavastatin, a salt thereof or a solvate thereof as an HMG-CoA reductase inhibitor, and using it in combination with pemafibrate or a salt thereof.

Thus, the combination of pemafibrate or a salt thereof and the HMG-CoA reductase inhibitor of the present invention exhibits an excellent risk reducing effect against hypertension, dyslipidemia, and obesity, which are major risk factors for cardiovascular diseases, and is therefore useful as a prophylactic and/or therapeutic agent for cardiovascular diseases.

As used herein, "cardiovascular diseases" include heart and circulatory system disorders including coronary artery disease, cerebrovascular disease, peripheral artery disease, rheumatic heart disease, congenital heart disease, deep vein thrombosis and pulmonary embolism. Coronary artery disease occurs when the coronary arteries which supply oxygen and nutrients to the heart become narrowed or clogged, reducing blood flow to the myocardium. Angina, in which transient chest pain occurs, caused by insufficient blood flow due to the stenosis of the coronary arteries, and myocardial infarction, in which the disease further progresses, causing the coronary arteries to become occluded, or extremely narrowed and almost occluded, and the myocardium to undergo necrosis, are known. In addition, cerebrovascular disease is a disease caused by damage to the blood vessels in the brain, and is classified into cerebral hemorrhage (hemorrhagic cerebrovascular disorder) and cerebral infarction (ischemic cerebrovascular disorder). Among these, cerebral infarction is a disease in which the blood vessels in the brain are occluded by a thrombus, arteriosclerosis, or the like, and the cells ahead of them become necrotic.

The combination ratio of pemafibrate or a salt thereof and the HMG-CoA reductase inhibitor is not limited, and can be appropriately examined and determined according to the sex, age, symptoms, and the like of the recipient. From the viewpoint of obtaining an excellent cardiovascular risk reducing effect, it is preferable to combine from 0.001 to 160,000 parts by mass, more preferable to combine from 0.05 to 12,000 parts by mass, and particularly preferable to combine from 2.5 to 800 parts by mass of the HMG-CoA reductase inhibitor in terms of the free form with 1 part by mass of the free form of pemafibrate. In particular, when using pitavastatin, a salt thereof or a solvate thereof as the HMG-CoA reductase inhibitor, from the viewpoint of obtaining an excellent cardiovascular risk reducing effect, it is preferable to combine 0.001 to 16,000 parts by mass, more preferable to combine 0.05 to 800 parts by mass, and particularly preferable to combine 0.6 to 40 parts by mass of pitavastatin, a salt thereof or a solvate thereof in terms of the free form with 1 part by mass of the free form of pemafibrate.

The prophylactic and/or therapeutic agent for cardiovascular diseases, obtained by combining pemafibrate or a salt thereof with the HMG-CoA reductase inhibitor, may be administered simultaneously or at an interval as separate and independent preparations of pemafibrate or a salt thereof and the HMG-CoA reductase inhibitor, which are the components (for example, in the form of a single package (kit preparation) containing a combination of a preparation containing pemafibrate or a salt thereof and a preparation containing the HMG-CoA reductase inhibitor), or it may be administered as a pharmaceutical preparation (combination drug) containing both components. However, it is preferable to use a combination drug containing both components from the viewpoint of ease of administration.

The prophylactic and/or therapeutic agent for cardiovascular diseases containing an HMG-CoA reductase inhibitor administered in combination with pemafibrate or a salt thereof, is intended to be used in combination with pemafibrate or a salt thereof. For example, a medicament containing the HMG-CoA reductase inhibitor is suitably administered simultaneously or at an interval with a medicament containing pemafibrate or a salt thereof.

A specific aspect of the combination is, for example, a pharmaceutical product for preventing and/or treating cardiovascular diseases, which contains the following (A) and (B):
(A) a medicament for preventing and/or treating cardiovascular diseases, which contains the HMG-CoA reductase inhibitor;
(B) instructions for administering the medicament in combination with pemafibrate or a salt thereof.

Specific examples of the instructions include a so-called package insert (an attached document), label, or the like, which contains the explanations regarding indication, dosage and administration, and the like.

In addition, the prophylactic and/or therapeutic agent for cardiovascular diseases containing pemafibrate or a salt thereof administered in combination with the HMG-CoA reductase inhibitor, is intended to be used in combination with the HMG-CoA reductase inhibitor. For example, a medicament containing pemafibrate or a salt thereof is suitably administered simultaneously or at an interval with a medicament containing the HMG-CoA reductase inhibitor.

A specific aspect of the medicament is, for example, a pharmaceutical product for preventing and/or treating cardiovascular diseases, which contains the following (A) and (B):
(A) a medicament for preventing and/or treating cardiovascular diseases, which contains pemafibrate or a salt thereof;
(B) instructions for administering the medicament in combination with the HMG-CoA reductase inhibitor.

Specific examples of the instructions include a so-called package insert (an attached document), label, or the like, which contains the explanations regarding indication, dosage and administration, and the like.

In the present description, the specific form (dosage form) of the medicament is not limited. It may be any of a solid, semi-solid, or liquid preparation form, and can be selected according to the purpose of use and the like. Examples of the dosage form of the medicament include the dosage forms described in the Japanese Pharmacopoeia 16th Edition, General Rules for Preparations and the like. More specifically, the dosage forms for oral administration include solid preparations such as tablets (including, for example, normal tablets, orally disintegrating tablets, chewable tablets, effervescent tablets, dispersible tablets, dissolving tablets, and the like), capsules, granules (including, for example, effervescent granules and the like), and powders; semi-solid preparations such as oral jelly; and liquid preparations such as oral liquids (including, for example, elixirs, suspensions, emulsions, limonades and the like). In addition, the dosage forms for parenteral administration include injections, inhalants, eye drops, ear drops, nasal drops, suppositories, external solid preparations, external liquid preparations, sprays, ointments, creams, gels, and patches.

From the viewpoint of ease of administration, the dosage form of the medicament is preferably a solid preparation for oral administration, particularly preferably tablets, capsules, granules or powders.

The medicament can be produced by a known method described in, for example, Japanese Pharmacopoeia 16th Edition, General Rules for Preparation, according to the dosage form. In this case, a pharmaceutically acceptable carrier (additive) may be added to the medicament. Examples of such additives include, but are not limited to, excipients, binders, fillers, disintegrants, surfactants, lubricants, dispersants, buffers, preservatives, flavoring agents, fragrances, coating agents, and diluents.

### EXAMPLES

Hereinafter, the present invention will be further described with reference to the Examples. However, the present invention is not limited to these Examples.

### Example 1. Effects on blood pressure, heart weight, and vascular endothelial function in Dahl salt-sensitive rats (Experimental method)

Drug efficacy was evaluated on Dahl salt-sensitive rats (Dahl-Iwai S, 7 weeks old, Japan SLC, Inc.) fed on a 8% salt-containing HFD (High Fat Diet: 29.4% fat content, CLEA Japan, Inc.) (4 groups in total: (1) control group, (2) pemafibrate (0.5 mg/kg/day) single-agent administration group, (3) pitavastatin (0.3 mg/kg/day) single-agent administration group, and (4) pemafibrate (0.5 mg/kg/day) + pitavastatin (0.3 mg/kg/day) combined administration group, with 10 animals for each group). Twelve weeks after the start of administration, the animals were euthanized after measuring their blood pressure, and the weight of the isolated hearts and the endothelial function of the isolated thoracic aortas were measured with a Magnus apparatus. The measurement with the Magnus apparatus was performed according to the standard method. That is, a ring preparation of the isolated thoracic aorta was prepared, suspended in a Magnus tube in Krebs-Henseleit solution, loaded with a tension of 1 g, and constricted with 0.3 µM phenylephrine. Then, acetylcholine (10⁻⁹ to 10⁻⁵ M) was added cumulatively, and the relaxation response was measured. Finally, 100% relaxation was obtained with 100 µM papaverine, and the relaxation rate was evaluated. When measuring with the Magnus apparatus, the blood vessels of normal rats were also evaluated in addition to the four groups, and were used as a control group. In addition, the expression level of phosphorylated endothelial nitric oxide synthase (phospho-eNOS) was evaluated by Western blotting according to the standard method, using the isolated aortas.

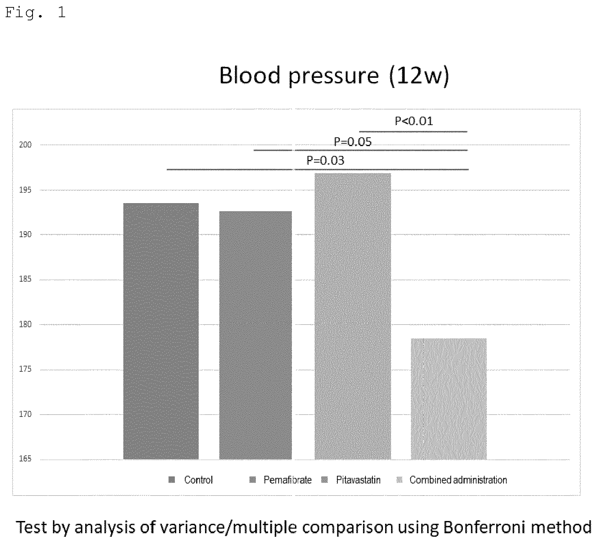

The results are shown in Figures 1 to 4. As shown in Figure 1, 12 weeks after the start of the test, the mean blood pressure of the control group was 193.6 ± 6.6 mmHg (mean ± standard deviation, the same applies hereinafter) due to 0.8% salt and high fat diet loading. By comparison, no change was observed in the pemafibrate single-administration group (mean blood pressure: 192.6 ± 8.3 mmHg) or the pitavastatin single-administration group (mean blood pressure: 196.9 ± 8.4 mmHg). In contrast, a significant decrease in blood pressure was observed in the combined administration group of both agents (mean blood pressure: 178.5 ± 10.1 mmHg) (analysis of variance/multiple comparison test by Bonferroni method).

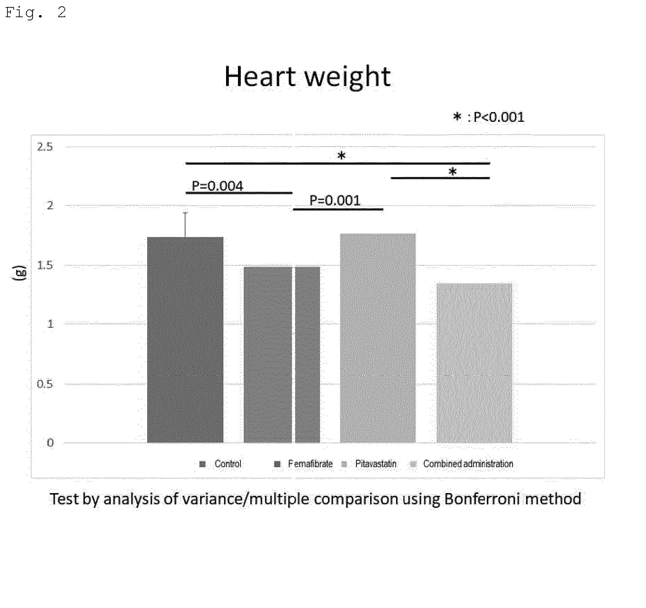

In addition, as shown in Figure 2, 12 weeks after the start of the test, the heart weight of the control group was 1.74 ± 0.1 g (mean±standard deviation, the same applies hereinafter) due to 0.8% salt and high fat diet loading. By comparison, the heart weight of the pemafibrate single administration group was 1.48 ± 0.1 g, which was significantly lower than that of the control. In contrast, the heart weight of the pitavastatin single administration group was 1.76 ± 0.2 g, showing no change compared to the control. In the combined administration group of both agents, the heart weight was 1.34 ± 0.1 g, and a significant decrease in blood pressure was observed compared to the other three groups including the pemafibrate single administration group (analysis of variance/multiple comparison test by Bonferroni method).

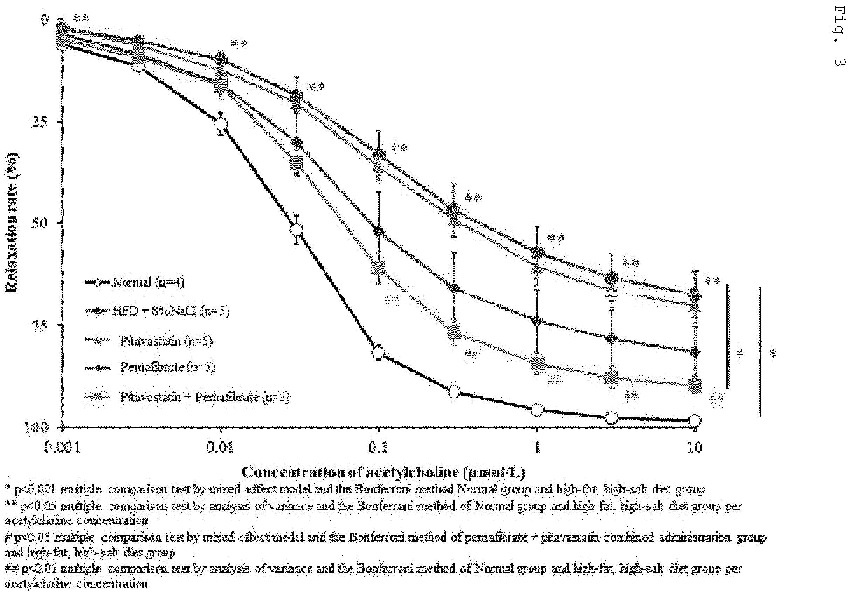

Furthermore, as shown in Figure 3, the vasodilatory response of the control group was significantly (multiple comparison test by mixed effect model and the Bonferroni method) weak as a whole, compared to the Normal group, and was significantly (analysis of variance/multiple comparison test by Bonferroni method) weak even at each measurement point, indicating a decrease in endothelial function. By comparison, the pitavastatin single administration group and the pemafibrate single administration group showed a trend toward improvement in vasodilatory responsiveness. However, no statistically significant difference was observed. However, the combined administration group of both agents significantly (multiple comparison test by mixed effect model and the Bonferroni method) showed a trend toward improvement in vasodilatory responsiveness as a whole, and the measurement points higher than 100 µmol/L of acetylcholine significantly (analysis of variance/multiple comparison test by Bonferroni method) showed an improvement in vasodilatory response, compared with the control group.

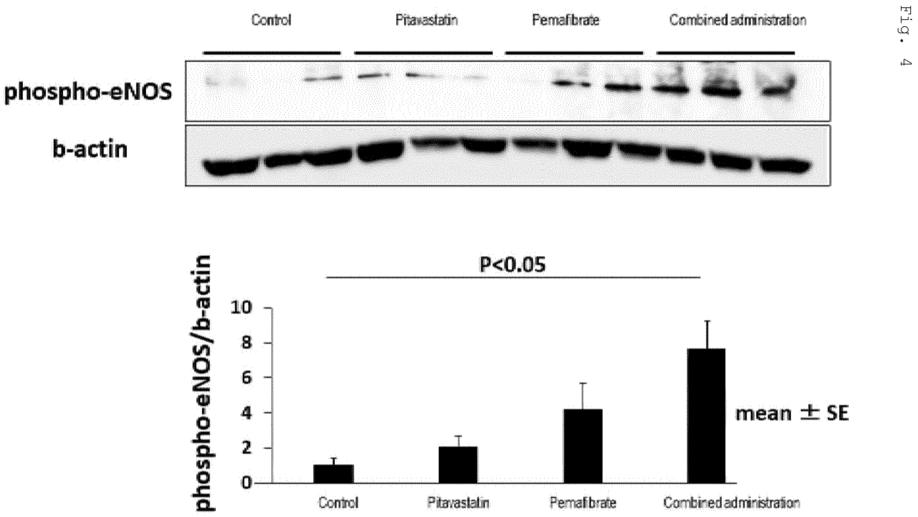

In addition, as shown in Figure 4, the expression level of phospho-eNOS showed a tendency to increase in both the pitavastatin single administration group and the pemafibrate single administration group, compared to the control group, and the combined administration group of both agents showed a significant increase compared to the control group (analysis of variance/multiple comparison test by Bonferroni method).

The above results revealed that the combined administration of pemafibrate and pitavastatin exhibits an effect of reducing blood pressure, suppressing increase in heart weight, and improving vascular endothelial function in Dahl salt-sensitive rats loaded with 0.8% salt and a high-fat diet, and therefore, that the medicament of the present invention is useful as a prophylactic and/or therapeutic agent for cardiovascular diseases.

### INDUSTRIAL APPLICABILITY

The medicament of the present invention is useful as a prophylactic and/or therapeutic agent for cardiovascular diseases, and thus has industrial applicability.

## Claims

1. A combination of pemafibrate or a salt thereof and pitavastatin, a salt thereof or a solvate thereof for use in reducing hypertension as a major risk factor for cardiovascular diseases.

2. The combination of pemafibrate or a salt thereof and pitavastatin, a salt thereof or a solvate thereof for the use according to claim 1, wherein the use is in reducing hypertension, dyslipidemia, and obesity as major risk factors for cardiovascular diseases.

3. The combination for use according to Claim 1 or 2, wherein the pitavastatin, a salt thereof or a solvate thereof is pitavastatin calcium or pitavastatin calcium hydrate.

## Patentansprüche

1. Eine Kombination aus Pemafibrat oder einem Salz davon und Pitavastatin, einem Salz davon oder einem Solvat davon zur Verwendung bei der Senkung von Bluthochdruck als einem Hauptrisikofaktor für Herz-Kreislauf-Erkrankungen.

2. Die Kombination von Pemafibrat oder einem Salz davon und Pitavastatin, einem Salz davon oder einem Solvat davon zur Verwendung gemäß Anspruch 1, wobei die Verwendung zur Verringerung von Bluthochdruck, Dyslipidämie und Adipositas als Hauptrisikofaktoren für Herz-Kreislauf-Erkrankungen dient.

3. Die Kombination zur Verwendung gemäß Anspruch 1 oder 2, wobei das Pitavastatin, ein Salz davon oder ein Solvat davon Pitavastatin-Calcium oder Pitavastatin-Calciumhydrat ist.

## Revendications

1. Combinaison de pémafibrate ou d'un sel de celui-ci et de pitavastatine, d'un sel de celle-ci ou d'un solvate de celle-ci pour une utilisation dans la réduction de l'hypertension en tant que facteur de risque majeur de maladies cardiovasculaires.

2. Combinaison de pémafibrate ou d'un sel de celui-ci et de pitavastatine, d'un sel de celle-ci ou d'un solvate de celle-ci pour l'utilisation selon la revendication 1, dans laquelle l'utilisation est dans la réduction de l'hypertension, de la dyslipidémie et de l'obésité en tant que facteurs de risque majeurs de maladies cardiovasculaires.

3. Combinaison pour l'utilisation selon la revendication 1 ou 2, dans laquelle la pitavastatine, un sel de celle-ci ou un solvate de celle-ci est la pitavastatine calcique ou l'hydrate de pitavastatine calcique.
